# EUROPÄISCHE PATENTSCHRIFT

(11) **EP 0 583 682 B1**
(45) Veröffentlichungstag und Bekanntmachung des Hinweises auf die Patenterteilung: **26.05.1999**
(21) Anmeldenummer: 93112465.5
(22) Anmeldetag: 04.08.1993
(51) Int. Cl.: C07D 277/66

(54) **Verfahren zur Herstellung von 2-(4-Aminophenyl)-benzothiazolverbindungen**
Process for the preparation of 2-(4-Aminophenyl)-benzothiazole derivatives
Procédé de préparation de dérivés de 2-(4-Aminophényl)-benzothiazoles

(30) Priorität: 14.08.1992 DE 4227029
(43) Veröffentlichungstag der Anmeldung: 23.02.1994
(73) Patentinhaber: BASF Aktiengesellschaft, 67063 Ludwigshafen (DE)
(72) Erfinder: Bomba, Christoph, Dr., D-6800 Mannheim 1 (DE); Kuth, Guido, Dr., D-6700 Ludwigshafen (DE); Guenthert, Paul, Dr., D-6707 Schifferstadt (DE); Hahn, Erwin, Dr., D-6900 Heidelberg (DE)

(56) Entgegenhaltungen:
- DE-A- 2 106 200
- DE-A- 2 333 378
- CHEMICAL ABSTRACTS, vol. 105, no. 21, 24. November 1986, Columbus, Ohio, US; abstract no. 191069y, Seite 728 ; & CS-A-223 275 (KAREL PASTALKA ET AL) 15. März 1986

## Beschreibung

Die vorliegende Erfindung betrifft ein neues Verfahren zur Herstellung von Verbindungen der allgemeinen Formel I 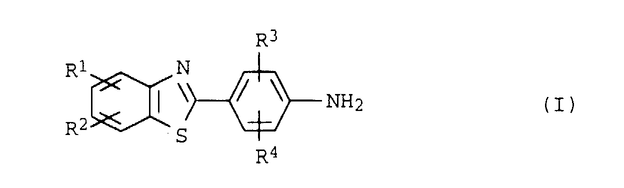 in der R¹ bis R⁴ unabhängig voneinander Wasserstoff, C₁-C₆-Alkyl, C₁-C₆-Alkoxy, Hydroxy oder Halogen bedeuten.

Es ist bekannt, daß substituierte 2-(4-Aminophenyl)-benzthiazole durch Kondensation von o-Aminothiophenolen mit aromatischen Aminen in Gegenwart eines Oxidationsmittels sowohl unter Verwendung eines Lösungsmittels [z.B. nach A.W. Hoffmann, Ber. 13 (1880), 1236; M.T. Bogert und A. Stull, J. Am. Chem. Soc. 47 (1925), 3078; H. Lankelma und P.J. Sharnoff, J. Am. Chem. Soc. 53 (1931), 2654 und 54 (1932), 379; US-A-3 772 309; DE-A-2 333 378 oder T.G. Deligeorgiev, Dyes and Pigments 12 (1990), 243] als auch ohne Lösungsmittel [z.B. nach A.I. Kiprianov und V.A. Shrubovich, Zh. Obshch. Khim. 30 (1960), 3746] hergestellt werden können.

Ferner ist aus Ullmanns Encyklopädie der technischen Chemie, 3. Auflage, Bd. 17 (1966), Seiten 333-334 die Herstellung von Dehydrothiotoluidin bekannt, bei der ein Gemisch von p-Toluidin, Schwefel und Natriumcarbonat auf Temperaturen von zunächst 180°C und später bis zu 222°C erhitzt wird. Die Ausbeute an gereinigtem Dehydrothiotoluidin beträgt 57 % d. Th.

Nunmehr wurde ein Verfahren zur Herstellung von Verbindungen der allgemeinen Formel I 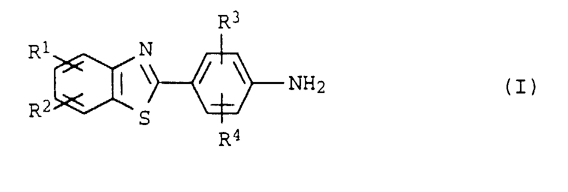 in der R¹ bis R⁴ unabhängig voneinander Wasserstoff, C₁-C₆-Alkyl, C₁-C₆-Alkoxy, Hydroxy oder Halogen bedeuten, gefunden, welches dadurch gekennzeichnet ist, daß man einen Überschuß von Verbindungen der Formel II 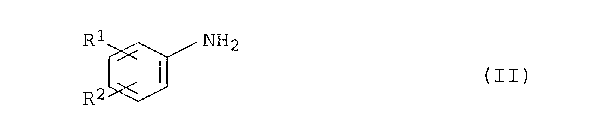 mit Verbindungen der Formel III 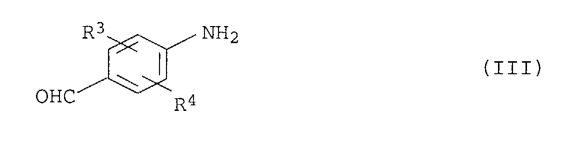 in Gegenwart von Schwefel oder schwefelabgebenden Verbindungen bei erhöhter Temperatur, bei der die Mischung aus Verbindungen II und Schwefel flüssig ist, umsetzt.

Als Substituenten R¹ bis R⁴ sind dabei neben den bereits einzeln genannten Fluor, Chlor oder Brom sowie C₁- bis C₆-Alkyl- oder -Alkoxyreste wie Methyl, Ethyl, n- oder i-Propyl, n-, i- oder tert.-Butyl, Hexyl, Methoxy, Ethoxy oder Butoxy aufzuführen. Vorzugsweise ist R¹ Methyl und R² bis R⁴ stehen für Wasserstoff.

Die erfindungsgemäße Umsetzung wird zweckmäßigerweise so vorgenommen, daß man in das flüssig vorliegende Gemisch aus der Verbindung der Formel II und Schwefel allmählich die Verbindung der Formel III einträgt.

In der Regel führt man die Umsetzung bei Temperaturen zwischen 150 und 230, vorzugsweise 170 bis 190°C, durch. Es ist dabei vorteilhaft, die Verbindung der Formel III in Form der Azomethine zu verwenden, wobei als Aminkomponente vorzugsweise Verbindungen der Formel II verwendet werden.

Besonders bevorzugt beim erfindungsgemäßen Verfahren ist die Zugabe eines Gemisches aus den Verbindungen der Formel III und II im Verhältnis 0,6 bis 1,0, wobei die Verbindung der Formel III auch ganz oder teilweise in Form des Azomethins vorliegen kann.

Verbindungen der Formel III können z.B. nach dem in der US-A-2 795 614 beschriebenen Verfahren hergestellt werden.

Verbindungen der Formel I, worin R¹ 6-Methyl und R² - R⁴ Wasserstoff bedeuten können nach den in DE-A-2 106 200 und CS-A-0 223 275 beschriebenen Verfahren hergestellt werden.

### Beispiel 3

Zu einer Schmelze aus 100 Teilen p-Anisidin und 11 Teilen Schwefel wurden analog Beispiel 1 17 Teile p-Aminobenzaldehyd zugegeben. Die nach Abtrennung von p-Anisidin erhaltene Rohschmelze enthielt ungefähr 80 % 2-(4-Aminophenyl)-6-methoxy-benzthiazol.

Das erfindungsgemäße Verfahren liefert die Verbindungen der Formel I nach Abtrennung des Überschusses der Verbindung der Formel II in vorzüglicher Ausbeute und Reinheit. Üblicherweise werden die Verbindungen der Formel II in 2- bis 12-fachem, vorzugsweise in 4- bis 8-fachem molarem Überschuß, bezogen auf die Verbindungen der Formel III, verwendet. Schwefel wird zweckmäßigerweise in 1,5- bis 3,5-fachem, vorzugsweise in 2,25- bis 2,75-fachem Überschuß verwendet.

Nach beendeter Reaktion können die Verbindungen der Formel II durch Abdestillieren entfernt werden. Die Verbindungen der Formel I können z.B. durch Umkristallisieren oder Feststoffdestillation gereinigt werden, es ist aber auch möglich, sie aufgrund ihrer hohen Reinheit direkt für weitere Umsetzungen, beispielsweise die Sulfierung oder Diazotierung zu verwenden.

Verbindungen der Formel I sind mit oder ohne Sulfogruppe wertvolle Diazokomponenten.

Einzelheiten des erfindungsgemäßen Verfahrens können den Beispielen entnommen werden, in denen sich Angaben über Teile und Prozente, sofern nicht anders vermerkt, auf Gewicht beziehen.

### Beispiel 1

Zu einer Schmelze aus 100 Teilen p-Toluidin und 12 Teilen Schwefel wurden bei einer Innentemperatur von 175 bis 180°C 43 Teile eines p-Aminobenzaldehyd/p-Toluidin-Gemisches (Molverhältnis ca. 0,65) über einen Zeitraum von sechs Stunden kontinuierlich oder portionsweise zugegeben. Nach beendeter Reaktion wurde das überschüssige p-Toluidin destillativ entfernt. Die gewonnene Rohschmelze (ca. 47 Teile) enthielt ungefähr 95 % 2-(4-Aminophenyl)-6-methylbenzthiazol (Dehydrothiotoluidin). Sie konnte ohne weitere Reinigung sulfiert und/oder diazotiert und dann zu wertvollen Papierfarbstoffen umgesetzt werden.

### Beispiel 2

Zu einer Schmelze aus 100 Teilen Anilin und 14 Teilen Schwefel wurden analog Beispiel 1 24 Teile p-Aminobenzaldehyd zugegeben. Die nach dem Entfernen des Anilins erhaltene Rohschmelze enthielt ungefähr 80 % 2-(4-Aminophenyl)benzthiazol.

## Patentansprüche

1. Verfahren zur Herstellung von Verbindungen der allgemeinen Formel I 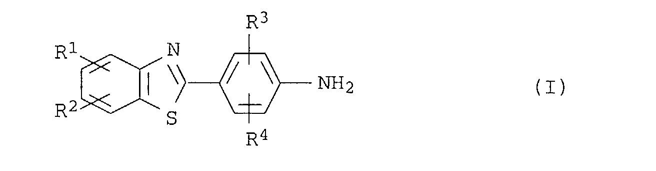 in der R¹ bis R⁴ unabhängig voneinander Wasserstoff, C₁-C₆-Alkyl, C₁-C₆-Alkoxy, Hydroxy oder Halogen bedeuten, dadurch gekennzeichnet, daß man einen Überschuß von Verbindungen der Formel II 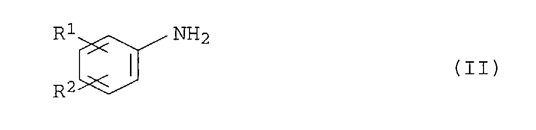 mit Verbindungen der Formel III 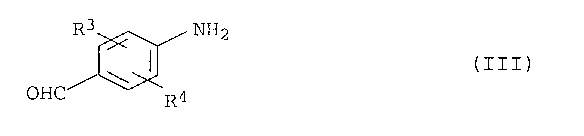 in Gegenwart von Schwefel oder schwefelabgebenden Verbindungen bei erhöhter Temperatur, bei der die Mischung aus Verbindungen II und Schwefel flüssig ist, umsetzt.

2. Verfahren nach Anspruch 1, dadurch gekennzeichnet, daß man die Verbindung der Formel II und Schwefel vorlegt und die Verbindung der Formel III allmählich zugibt.

3. Verfahren nach Anspruch 1 oder 2, dadurch gekennzeichnet, daß man die Verbindung der Formel III in Form eines Azomethins verwendet.

## Claims

1. A process for preparing a compound of the formula I 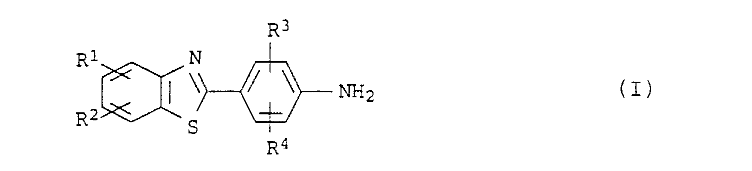 where R¹ to R⁴ are each, independently of one another, hydrogen, C₁-C₆-alkyl, C₁-C₆-alkoxy, hydroxyl or halogen, which comprises reacting an excess of a compound of the formula II 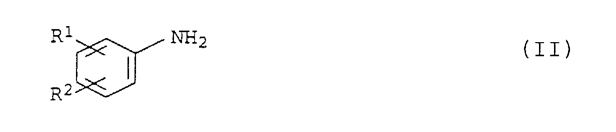 with a compound of the formula III 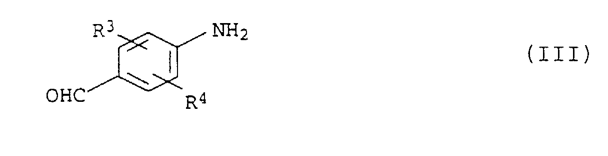 in the presence of sulfur or a sulfur donor at elevated temperature at which the mixture of compound II and sulfur is liquid.

2. A process as claimed in claim 1, wherein the compound of the formula III is gradually added to a mixture of the compound of the formula II and sulfur.

3. A process as claimed in claim 1 or 2, wherein the compound of the formula III is used in the form of an azomethine.

## Revendications

1. Procédé de préparation de composés de formule générale I 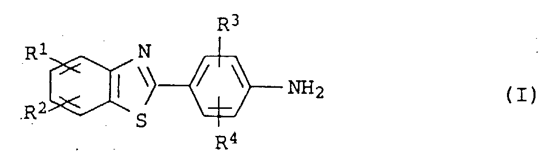 dans laquelle R¹ à R⁴ représentent indépendamment les uns des autres, un atome d'hydrogène, un groupement alkyle en C₁-C₆, un groupement alcoxy en C₁-C₆, un groupement hydroxy ou un atome d'halogène, caractérisé en ce que l'on fait réagir un excès de composés de formule II 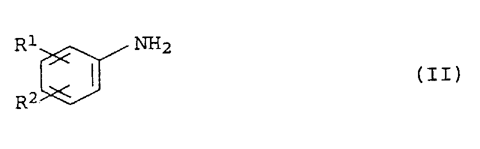 avec des composés de formule III 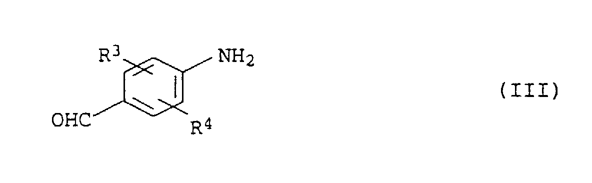 en présence de soufre ou de composés libérant du soufre à des températures élevées, auxquelles le mélange des composés II et du soufre est liquide.

2. Procédé selon la revendication 1, caractérisé en ce que l'on dispose au préalable le composé de formule II et le soufre puis l'on ajoute le composé de formule III de façon progressive.

3. Procédé selon la revendication 2 ou 2, caractérisé en ce que l'on utilise le composé de formule III sous forme d'un azométhine.
